# EUROPEAN PATENT APPLICATION

(11) **EP 4 534 000 A1**
(43) Date of publication of application: **09.04.2025**
(21) Application number: 23202081.8
(22) Date of filing: 06.10.2023
(51) Int. Cl.: A61B 5/00, A61B 5/021, A61B 5/024

(54) **SYSTEMS AND METHODS FOR SYNCHRONIZING HEALTH MONITORING DEVICES FOR ACCURATE PROCESSING OF SHARED SIGNALS**

(71) Applicant: Koninklijke Philips N.V., 5656 AG Eindhoven (NL)
(72) Inventor: DE JAGER, Marinus Karel Johannes, Eindhoven (NL); GELISSEN, Jozef Hubertus, Eindhoven (NL); TEN KATE, Warner Rudolph Theophile, 5656AG Eindhoven (NL); VALENTI, Giulio, Eindhoven (NL); BRANDSMA, Ewout, Eindhoven (NL); HUIJGEN, Henk, Eindhoven (NL)
(74) Representative: Philips Intellectual Property & Standards

(57) **Abstract**

Health monitoring systems and methods configured to synchronize separate monitoring devices for accurate processing of shared signals are provided. While the use of health monitoring devices is increasing in popularity and a growing number of devices are becoming available, each of these devices can have different manufacturers and operating specifications. As such, data collected across devices cannot be readily integrated. In accordance with the present disclosure, the systems and methods provided enable the synchronization of multiple health monitoring devices using only a physiological signal measured by two or more of the devices.

## Description

### FIELD OF THE DISCLOSURE

The present disclosure relates generally to systems and methods of utilizing multiple physiological signals across different health monitoring devices, and more specifically to systems and methods of synchronizing physiological signals collected across different monitoring devices.

### BACKGROUND

In recent years, there has been significant growth in the popularity of wearable electronics, including electronics designed to be worn on the body and monitor various health metrics, such as heart rate, steps taken, sleep patterns, and the like. Advancements in this field have made these devices smaller, more comfortable, more affordable, and more accurate. As a result, these devices have become increasingly common as people become more interested in tracking and improving their health. Additionally, it has become increasingly common that an individual may utilize more than one such device at a time, such as a smartphone that tracks a user's steps and a smartwatch that tracks the user's heart rate. Other devices that have recently become popular include various patches, chest straps, bracelets and wristbands, rings, smart garments, implantable cardiac monitors, and the like, which have greatly expanded the possible options for health monitoring regimens that can be performed using combinations of separate devices, typically not in communication or synchronized with each other.

### SUMMARY OF THE DISCLOSURE

Important health-related information can be collected and analyzed using a variety of health monitoring devices, including various wearable devices such as patches, chest straps, bracelets and wristbands, rings, and the like. Because of the increasing popularity of such devices, it is also possible to integrate health-related information across different devices. However, synchronizing signals collected by these different devices, which are often sold under different brands and have different manufacturers and operating specifications, is a significant challenge. For example, each of these devices have their own operating clocks that run unsynchronized. Thus, the present disclosure is directed to systems and methods of synchronizing two or more separate monitoring devices for accurate processing of shared signals.

According to an embodiment of the present disclosure, a health monitoring system configured to synchronize separate monitoring devices for accurate processing of shared signals is provided. The system may include: a computer-readable storage medium having stored thereon machine-readable instructions to be executed by one or more processors; and one or more processors configured by the machine-readable instructions stored on the computer-readable storage medium to perform the following operations: (i) obtain a first set of physiological signals from a first monitoring device and a second monitoring device, wherein the first set of physiological signals includes a first physiological signal from the first monitoring device covering a first time period and a second physiological signal from the second monitoring device covering at least a portion of the first time period; (ii) analyze at least the first and second physiological signals of the first set of physiological signals to determine a first time marker; (iii) obtain a second set of physiological signals from the first monitoring device and the second monitoring device, wherein the second set of physiological signals includes a first physiological signal from the first monitoring device covering a second time period and a second physiological signal from the second monitoring device covering at least a portion of the second time period; (iv) analyze at least the first and second physiological signals of the second set of physiological signals to determine a second time marker; and (v) synchronize the first and second monitoring devices based on the first and second time markers.

In an aspect, the first time marker may be determined by correlating one or more signal features of the first and second physiological signals of the first set of physiological signals, the first time marker being indicative of when a shared signal feature is observed in the first and second physiological signals of the first set of physiological signals.

In an aspect, the second time marker may be determined by correlating one or more signal features of the first and second physiological signals of the second set of physiological signals, the second time marker being indicative of when a shared signal feature is observed in the first and second physiological signals of the second set of physiological signals.

In an aspect, the first and second physiological signals of the first set of physiological signals may be electrocardiogram signals, and the first and second physiological signals of the second set of physiological signals may be electrocardiogram signals.

In an aspect, synchronizing the first and second monitoring devices can include: (i) setting a leading anchor point for at least one physiological signal collected by the second monitoring device and a leading anchor point for at least one physiological signal collected by the first monitoring device, wherein the leading anchor points are set based on the first time marker; (ii) setting a trailing anchor point for the at least one physiological signal collected by the second monitoring device and a trailing anchor point for the at least one physiological signal collected by the first monitoring device, wherein the trailing anchor points are set based on the second time marker; and (iii) generating a synchronized physiological signal by resampling the at least one physiological signal collected by first monitoring device based on a relationship between the leading and trailing anchor points.

In an aspect, the one or more processors can be further configured by the machine-readable instructions stored on the computer-readable storage medium to perform the following operations: (i) obtain at least a third physiological signal from the second monitoring device; and (ii) analyze the third physiological signal and the synchronized physiological signal to determine a physiological measurement of interest.

In an aspect, the third physiological signal can be a photoplethysmography signal, the synchronized physiological signal can be either a photoplethysmography signal or an electrocardiogram signal, and the physiological measurement of interest is at least one of a pulse arrival time and a pulse transit time.

In an aspect, the health monitoring system can further include: a first monitoring device and a second monitoring device in communication with the one or more processors, wherein the first monitoring device is configured to measure one or more physiological signals including at least a first signal type and the second monitoring device is configured to measure one or more physiological signals including at least the first signal type.

According to another embodiment of the present disclosure, a non-transitory computer-readable storage medium having stored thereon machine-readable instructions is provided. When executed by one or more processors, the machine-readable instructions cause the one or more processors to perform operations comprising: (i) obtaining a first set of physiological signals from a first monitoring device and a second monitoring device, wherein the first set of physiological signals includes a first physiological signal from the first monitoring device covering a first time period and a second physiological signal from the second monitoring device covering at least a portion of the first time period; (ii) analyzing at least the first and second physiological signals of the first set of physiological signals to determine a first time marker; (iii) obtaining a second set of physiological signals from the first monitoring device and the second monitoring device, wherein the second set of physiological signals includes a first physiological signal from the first monitoring device covering a second time period and a second physiological signal from the second monitoring device covering at least a portion of the second time period; (iv) analyzing at least the first and second physiological signals of the second set of physiological signals to determine a second time marker; and (v) synchronizing the first and second monitoring devices based on the first and second time markers.

According to another embodiment of the present disclosure, a computer-implemented method for synchronizing multiple monitoring devices in a health monitoring system is provided. The method can include: obtaining a first set of physiological signals from a first monitoring device and a second monitoring device, wherein the first set of physiological signals includes a first physiological signal from the first monitoring device covering a first time period and a second physiological signal from the second monitoring device covering at least a portion of the first time period; analyzing at least the first and second physiological signals of the first set of physiological signals to determine a first time marker; obtaining a second set of physiological signals from the first monitoring device and the second monitoring device, wherein the second set of physiological signals includes a first physiological signal from the first monitoring device covering a second time period and a second physiological signal from the second monitoring device covering at least a portion of the second time period; analyzing at least the first and second physiological signals of the second set of physiological signals to determine a second time marker; and synchronizing the first and second monitoring devices based on the first and second time markers.

In an aspect, the first time marker can be determined by correlating one or more signal features of the first and second physiological signals of the first set of physiological signals, the first time marker being indicative of when a shared signal feature is observed in the first and second physiological signals of the first set of physiological signals.

In an aspect, the second time marker can be determined by correlating one or more signal features of the first and second physiological signals of the second set of physiological signals, the second time marker being indicative of when a shared signal feature is observed in the first and second physiological signals of the second set of physiological signals.

In an aspect, the first and second physiological signals of the first set of physiological signals may be electrocardiogram signals, and the first and second physiological signals of the second set of physiological signals may be electrocardiogram signals.

In an aspect, synchronizing the first and second monitoring devices can include: setting a leading anchor point for at least one physiological signal collected by the second monitoring device and a leading anchor point for at least one physiological signal collected by the first monitoring device, wherein the leading anchor points are set based on the first time marker; setting a trailing anchor point for the at least one physiological signal collected by the second monitoring device and a trailing anchor point for the at least one physiological signal collected by the first monitoring device, wherein the trailing anchor points are set based on the second time marker; and generating a synchronized physiological signal by resampling the at least one physiological signal collected by the first monitoring device based on a relationship between the leading and trailing anchor points.

In an aspect, the method can further include: obtaining a third physiological signal from the second monitoring device; and analyzing the third physiological signal and synchronized physiological signal to determine a physiological measurement of interest.

In an aspect, the third physiological signal may be a photoplethysmography signal, the synchronized physiological signal may be either a photoplethysmography signal or an electrocardiogram signal, and the physiological measurement of interest may be at least one of a pulse arrival time and a pulse transit time.

In an aspect, the health monitoring system may be a cloud-based system configured to wirelessly receive one or more physiological signals from the first and second monitoring devices.

These and other aspects of the various embodiments will be apparent from and elucidated with reference to the embodiments described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the various embodiments.
FIG. 1 is a diagram illustrating a health monitoring system configured to synchronize separate monitoring devices for accurate processing of shared signals according to aspects of the present disclosure.
FIG. 2 is a flowchart illustrating a computer-implemented method for synchronizing multiple monitoring devices in a health monitoring system according to aspects of the present disclosure.
FIG. 3 is a series of graphs illustrating the cross-correlation of two ECG signals according to aspects of the present disclosure.
FIG. 4 is a series of graphs illustrating the cross-correlation of two ECG signals according to further aspects of the present disclosure.
FIG. 5 is a flowchart illustrating a computer-implemented method for synchronizing multiple monitoring devices according to further aspects of the present disclosure.
FIG. 6 is a flowchart illustrating a computer-implemented method for synchronizing multiple monitoring devices according to still further aspects of the present disclosure.
FIG. 7 is a diagram illustrating the determination of a physiological measurement of interest based on a physiological signal collected from one device and a synchronized physiological signal collected from another device according to aspects of the present disclosure.
FIG. 8 is a diagram illustrating a health monitoring system according to aspects of the present disclosure.

### DETAILED DESCRIPTION OF EMBODIMENTS

The present disclosure is generally directed to health monitoring systems and methods that include at least two separate health monitoring devices, and more specifically to systems and methods that involve synchronizing physiological signals between separate devices for accurate processing of a combination of signals. For example, in one embodiment, a health monitoring system for non-invasive blood pressure monitoring is provided that includes at least a first wearable device positioned at the user's chest and configured to measure an electrocardiogram (ECG) signal, and at least a second wearable device positioned at the user's wrist or wait and configured to measure a photoplethysmogram (PPG) signal.

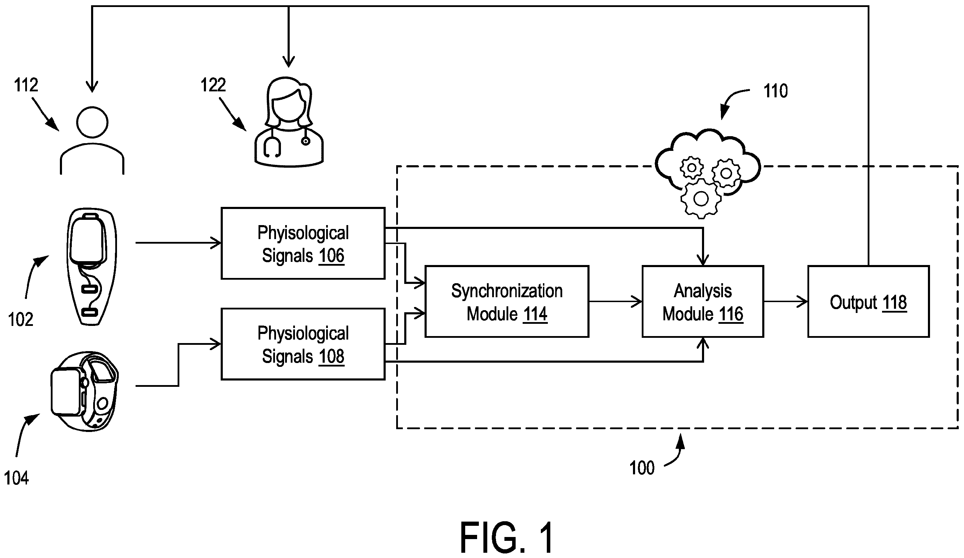

However, accurate blood pressure measurements require that these ECG and PPG signals be synchronized between the first and second wearable devices, which is a challenge because each device can have its own electronic clock determining sampling frequency and other relevant parameters. Accordingly, the health monitoring systems and methods of the present disclosure further involve the synchronization of multiple wearable devices using a shared physiological signal.
For example, with reference to FIG. 1, a health monitoring system 100 configured to synchronize separate monitoring devices 102, 104 for accurate processing of multiple signals 106, 108 is illustrated according to certain aspects of the present disclosure.

As shown, the health monitoring system 100 may be part of a cloud-based system 110 configured to receive one or more physiological signals 106, 108 from one or more wearable devices 102, 104. In further embodiments, the synchronization might be hosted on one or more of the devices 102, 104. That is, one or more of the devices 102, 104 may be configured to perform one or more steps of the synchronization methods described herein.

In particular embodiments, each device 102, 104 is configured to measure one or more physiological signals including at least one common signal type. For example, in some embodiments, the monitoring device 102 may be configured to measure an ECG signal, while the monitoring device 104 may be configured to measure an ECG signal as well as a PPG signal.

In specific embodiments, the monitoring device 102 is a patch placed at the user's (i.e., user 112) chest and configured to measure a continuous ECG signal, while the monitoring device 104 is a smartwatch worn on the user's wrist (i.e., user 112) and configured to measure a continuous PPG signal as well as a spot-check ECG signal. In such embodiments, the monitoring device 104 may have a PPG sensor disposed on the backside of the device and in contact with the user's (i.e., 112) skin, as well as ECG electrodes on opposing sides of the device 104 (i.e., one electrode touching the skin and one unconnected to skin), such that when the user 112 contacts that unconnected electrode with, e.g., the free hand (i.e., the one without the watch), a spot-check ECG signal can be collected. By using a continuous ECG signal 106 from the monitoring device 102 and a spot-check ECG signal 108, a synchronization operation may be performed to synchronize all of the physiological signals 106, 108 across the devices 102, 104.

More generally, in accordance with the present disclosure, multiple signals 106, 108 may be synchronized between the devices 102, 104 using at least one physiological signal 106, 108 common to both of the devices 102, 104. As shown in the example of FIG. 1, the health monitoring system 100 may include a synchronization module 114 configured to receive one or more physiological signals 106, 108 common to both devices 102, 104 and produce a synchronized physiological signal. Put another way, the output of the synchronization module 114 may be a mapping of two or more physiological signals 106, 108 from different devices 102, 104 to a common, synchronized time-base.

The health monitoring system 100 may also include an analysis module 116 configured to receive the synchronized physiological signal from the synchronization module 114 and one or more additional physiological signals 106, 108 from the devices 102, 104 and perform one or more operations on the received signals. For example, in some embodiments, the analysis module 116 may process the synchronized physiological signal and at least one other physiological signal 106, 108 to determine a physiological measurement of interest. As mentioned above, this physiological measurement of interest 118 can be, but is not limited to, at least one of a pulse arrival time and a pulse transit time. As shown in FIG. 1, the output 118 may optionally be fed back to one or more individuals, such as the end-user 112 and/or a physician 122 or other healthcare professional associated with the end-user 112.

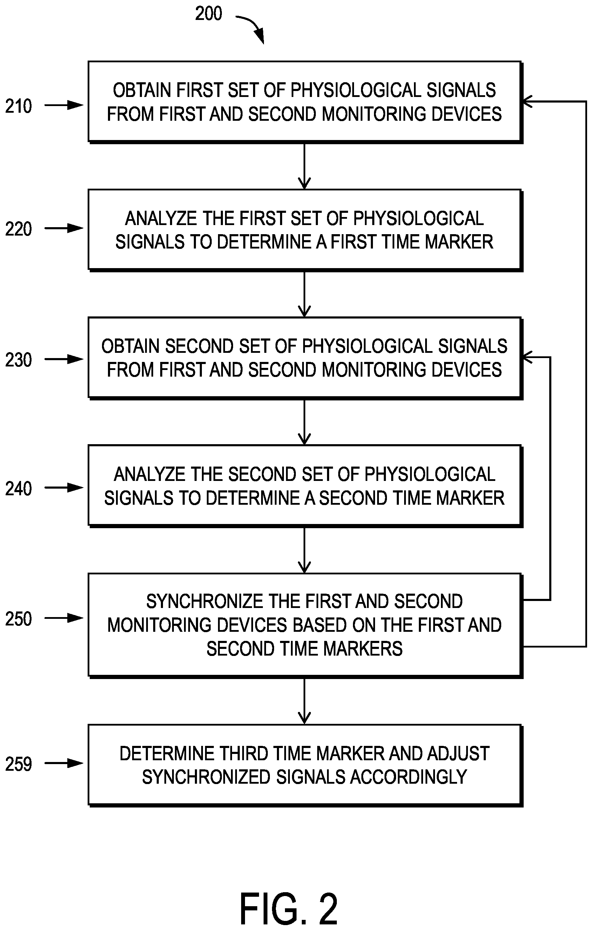

Turning to FIG. 2, a computer-implemented method 200 for synchronizing multiple monitoring devices 102, 104 in a health monitoring system 100 is illustrated according to certain aspects of the present disclosure.

The health monitoring system 100 can be any of the systems described or otherwise envisioned herein. As shown, the computer-implemented method 200 can include: in a step 210, obtaining a first set of physiological signals 106, 108 from at least two health monitoring devices 102, 104; in a step 220, analyzing the first set of physiological signal 106, 108 to determine a first time marker; in a step 230, obtaining a second set of physiological signals 106, 108 from at least the two health monitoring devices 102, 104; in a step 240, analyzing the second set of physiological signals 106, 108 to determine a second time marker; and in a step 250, synchronizing at least the two health monitoring devices 102, 104 based on the first and second time markers.

At the step 210, the computer-implemented method 200 includes obtaining a first set of physiological signals from a first monitoring device 102 and a second monitoring device 104, wherein the first set of physiological signals includes a first physiological signal 106 from the first monitoring device 102 covering a first time period and a second physiological signal 108 from the second monitoring device 104 covering at least a portion of the first time period.

As described herein, the first and second physiological signals 106, 108 of the first set of physiological signals can be common or shared signals. That is, in embodiments, the first and second physiological signals 106, 108 of the first set of physiological signals are the same type of physiological measurement but measured using two different devices 102, 104 positioned at different locations of the user 112.

In embodiments, the health monitoring devices 102, 104 can include one or more wearable health monitoring devices, including but not limited to, specialized medical devices such the Mobile Cardiac Outpatient Telemetry (MCOT) device provided by Koninklijke Philips N.V., as well as any number of commercial, off-the-shelf (COTS) devices. For example, the health monitoring devices 102, 104 can include one or more smart watches, rings, patches, straps (e.g., Holter monitor), textiles, implantable devices, and/or the like. As a non-exhaustive list, the health monitoring devices can include a Garmin^{®} watch (such as the Fenix 6, Vivoactive 4, and/or Venu 2 model watches), an Oura^{®} Ring, an Empatica^{™} embraced wristbands, an Apple^{®} Watch, a Samsung^{®} Galaxy Watch, a Google^{®} Pixel Watch, a Fitbit^{®} Charge, a Fitbit^{®} Versa, and/or the like. Notably, in particular embodiments, the first health monitoring device 102 is different and distinct from the second health monitoring device 104, meaning that each device 102, 104 operates on its own electronics, is separately housed, and is positioned on the user 112 at different locations.

It should be appreciated that physiological signals 106, 108 can be obtained from the one or more health monitoring devices 102, 104 in a variety of ways. For example, in particular embodiments, the physiological signals 106, 108 may be obtained directly from the health monitoring devices 102, 104 in real-time or near real-time over a wired and/or wireless connection. In other embodiments, the physiological signals 106, 108 may be obtained using an application programming interface (API) that communicates with a cloud-based service associated with the particular health monitoring devices 102, 104 being used by the individual. In some embodiments, a combination of these approaches may be utilized.

In embodiments, the physiological signals 106, 108 as well as any additional health-related information may be obtained over a secure protocol for data sharing and batch processed to convert any device- or source-specific data into a standardized data format (e.g., a compressed Python Pandas data frame format that standardizes column names for all inputs across devices). Various encryption and/or other data privacy measures may be implemented as part of obtaining the health-related information. Additionally, the information obtained in the step 210 may also include pertinent meta-data (e.g., timestamps, source identifiers, etc.).

As mentioned above, the first set of physiological signals can include a first physiological signal 106 from the first monitoring device 102 covering a first time period and a second physiological signal 108 from the second monitoring device 104 covering at least a portion of the first time period. In embodiments, the first time period can cover several seconds to several minutes, including a period of time up to the present time. Put another way, the first set of physiological signals can include real-time measurements taken over the first time period.

In particular embodiments, the first physiological signal 106 obtained via the first monitoring device 102 may be a continuous physiological signal 106 or a spot-check physiological signal 106, such as a continuous ECG signal or a spot-check ECG signal that covers at least a first time period. In further embodiments, the second physiological signal 108 obtained via the second monitoring device 104 may be a continuous physiological signal 108 or a spot-check physiological signal 108, such as a continuous ECG signal or spot-check ECG signal that covers at least a portion of the first time period. However, it should be appreciated that other types of shared physiological signals can be obtained, including but not limited to, PPG signals and (physiological) sounds.

In further embodiments, the first and second physiological signals 106, 108 may include physiological measurements taken during a particular activity or event. For example, in some embodiments, the first and second physiological signals 106, 108 may include physiological measurements taken while the user 112 takes deep breathes or lifts a weight. As a result, more distinctive signal features / landmarks may be observed in the following step 220.

At the step 220, the computer-implemented method 200 includes analyzing at least the first and second physiological signals 106, 108 of the first set of physiological signals to determine a first time marker. In embodiments, the first time marker is indicative of when a shared signal feature is observed in both the first and second physiological signals 106, 108 of the first set of physiological signals. Put another way, the step 220 includes correlating one or more signal features of the first and second physiological signals 106, 108 of the first set of physiological signals to identify the same moment in time, which is designated as the first time marker (i.e., *t*₀).

The signal features can include, for example, heartbeat intervals, but can also be the signal waveform itself (i.e., observable peaks, valleys, and/or patterns therein). In particular embodiments, the first and second physiological signals 106, 108 of the first set of physiological signals may be analyzed by comparing characteristic landmarks in the signals, such as the R-peak in the case that ECG signals are obtained. In embodiments, for example, R-peaks may be distinguished by their intrinsic varying rate and aligned peak by peak (e.g., by maximizing correlation between the signals). However, it should be appreciated that other signal features / landmarks may be utilized depending on the type of signal obtained from the first and second devices 102, 104.

In particular embodiments, correlating the first and second physiological signals 106, 108 can also include applying one or more signal processing techniques, such as detrending and/or high-pass filtering. In certain embodiments, the step 220 may include a pre-processing step whereby the physiological signals 106, 108 are resampled to a nominally equal sampling rate.

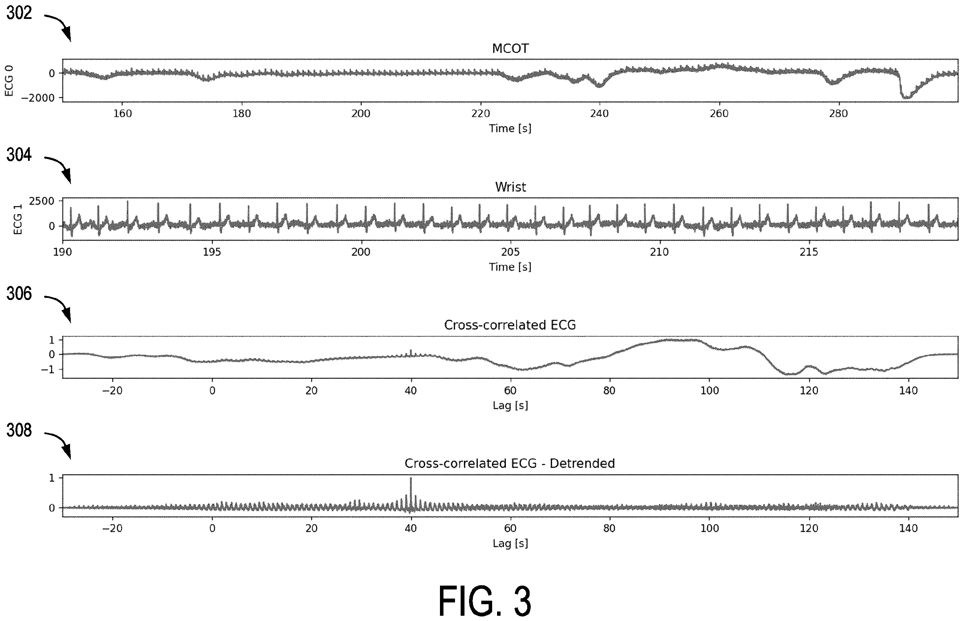

In specific embodiments, by comparing these landmarks / signal features, a lag time between each of the first and second physiological signals 106, 108 can be determined. For example, with reference to FIG. 3, an ECG signal 302 measured using an MCOT device (i.e., a chest patch device) and an ECG signal 304 measured using a wrist device are illustrated, along with a plot 306 of the signals' 302, 304 cross-correlation for various time lags, and a plot 308 of the same cross-correlation after applying a detrending step to it. Detrending can be achieved by applying a high-pass filter (on the series of cross-correlation values for various lags). As can be seen in the detrended plot 308, the largest correlation between the signals 302, 304 is found at a lag time of about 40 seconds. This indicates that the samples in signal 304 happening after 40 seconds according to the time base of 304 correspond in wall clock (i.e. physically) with the samples in signal 302 happening at current time of the time base of 302. In the example of FIG. 3, a high-pass filter was not applied to the ECG signals 302, 304. This would relax, if not remove, the need for the detrending step.

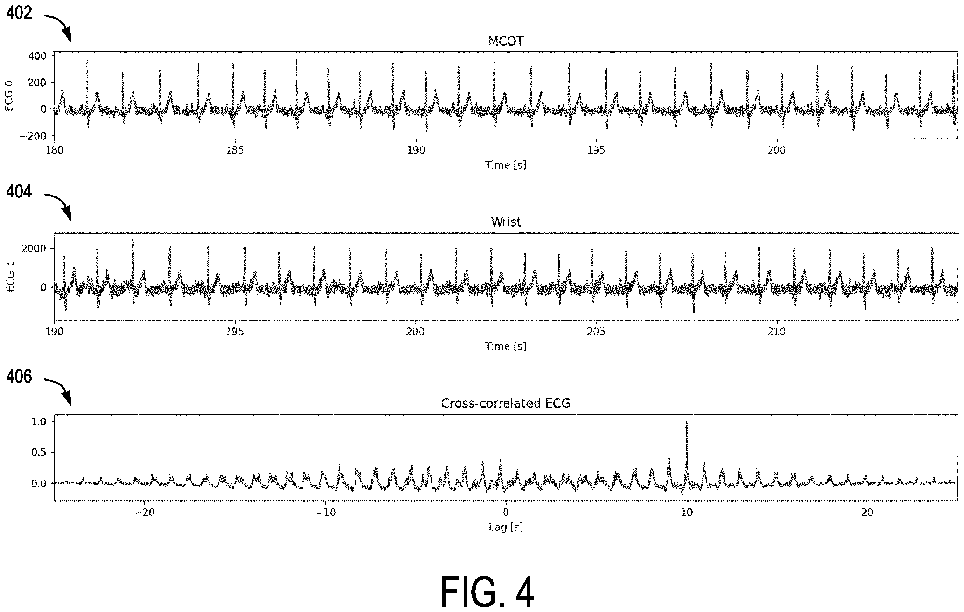

With reference to FIG. 4, an ECG signal 402 measured using another MCOT device and an ECG signal 404 measured using a wrist device are illustrated where a high-pass filter was applied, along with a cross-correlated ECG lag time plot 406. As shown, in this case the plot 406 indicates a high correlation between the signals 402, 404 at a lag time of about 10 seconds. This indicates that the samples in signal 404 happening after 10 seconds according to the time base of signal 404 correspond in wall clock (i.e. physically) with the samples in signal 402 happening at current time of the time base of 402. Those of ordinary skill in the art will appreciate that the direction of the shift (`happening after' or 'happening before' in both examples of FIG. 3 and FIG. 4) depends on the order at which the two signals are offered to the computation of the cross-correlation.

At the step 230, the computer-implemented method 200 includes obtaining a second set of physiological signals from the first monitoring device 102 and the second monitoring device 104, wherein the second set of physiological signals includes a first physiological signal 106 from the first monitoring device 102 covering a second time period and a second physiological signal 108 from the second monitoring device 104 covering at least a portion of the second time period.

In embodiments, the first and second physiological signals 106, 108 of the second set of physiological signals may be the same type or a different type of physiological signal as the first and second physiological signals 106, 108 of the first set of physiological signals. In particular embodiments, the first physiological signal 106 obtained via the first monitoring device 102 may be a continuous physiological signal 106 or a spot-check physiological signal 106, such as a continuous ECG signal or a spot-check ECG signal that covers at least a second time period. In further embodiments, the second physiological signal 108 obtained via the second monitoring device 104 may be a continuous physiological signal 108 or a spot-check physiological signal 108, such as a continuous ECG signal or spot-check ECG signal that covers at least a portion of the second time period. However, it should be appreciated that other types of shared physiological signals can be obtained, including but not limited to, PPG signals and (physiological) sounds.

As mentioned above, the second set of physiological signals can include a first physiological signal 106 from the first monitoring device 102 covering a second time period and a second physiological signal 108 from the second monitoring device 104 covering at least a portion of the second time period. In embodiments, the second time period can cover several seconds to several minutes, including a period of time up to the present time. Put another way, the second time period can include a real-time measurement. In particular embodiments, the second time period occurs some time after the first time period. For example, in some embodiments, the second time period covers a period of time beginning several minutes to several hours after the end of the first time period.

Similarly, the physiological signals 106, 108 of the second set of physiological signals can be obtained from the one or more health monitoring devices 102, 104 in a variety of ways. For example, in particular embodiments, the physiological signals 106, 108 may be obtained directly from the health monitoring devices 102, 104 in real-time or near real-time over a wired and/or wireless connection. In other embodiments, the physiological signals 106, 108 may be obtained using an application programming interface (API) that communicates with a cloud-based service associated with the particular health monitoring devices 102, 104 being used by the individual. In some embodiments, a combination of these approaches may be utilized, as described above.

In particular embodiments, the first and second physiological signals 106, 108 of the second set of physiological signals may include physiological measurements taken during a particular activity or event. For example, in some embodiments, the first and second physiological signals 106, 108 may include physiological measurements taken while the user 112 takes deep breathes or lifts a weight. As a result, more distinctive signal features / landmarks may be observed in the following step 240.

At the step 240, the computer-implemented method 200 includes analyzing at least the first and second physiological signals 106, 108 of the second set of physiological signals to determine a second time marker. In embodiments, the second time marker is indicative of when a shared signal feature is observed in both the first and second physiological signals 106, 108 of the second set of physiological signals. Put another way, the step 240 includes correlating one or more signal features of the first and second physiological signals 106, 108 of the second set of physiological signals to identify the same moment in time, which is designated as the second time marker (i.e., *t*₁).

In embodiments, the signal features can include, for example, heartbeat intervals, but can also include the signal waveform itself (i.e., observable peaks, valleys, and/or patterns therein). In some embodiments, the first and second physiological signals 106, 108 of the second set of physiological signals may be analyzed by comparing characteristic landmarks in the signals, such as the R-peak in the case that ECG signals are obtained. However, it should be appreciated that other signal features / landmarks may be utilized depending on the type of signal obtained from the first and second devices 102, 104.

In particular embodiments, the first and second physiological signals 106, 108 of the second set of physiological signals may be analyzed by comparing these landmarks / signal features to determine a lag time between each of the first and second physiological signals 106, 108 as described above and shown in FIG. 3 and FIG. 4. Additionally, as described above with respect to step 220, the step 240 may also include applying one or more signal processing techniques, such as detrending, filtering, and/or resampling to a nominally equal sampling rate.

At the step 250, the computer-implemented method 200 includes synchronizing the first and second monitoring devices 102, 104 based on the first and second time markers. In embodiments, because the first and second time markers are known for each of the devices 102, 104, a leading point in time (i.e., *t*₀) can be set based on the first time marker, a trailing point in time (i.e., *t*₁) can be set based on the second time marker, and one or more physiological signals 106, 108 may be adjusted such that data points across the devices 102, 104 directly correspond to one another (e.g., the n-th data point of one physiological signal 106 corresponds in time to the n-th data point of another physiological signal 108, and so on). In particular embodiments, the step 250 produces a synchronized physiological signal and/or a mapping of two or more physiological signals from two or more different devices to a common time-based.

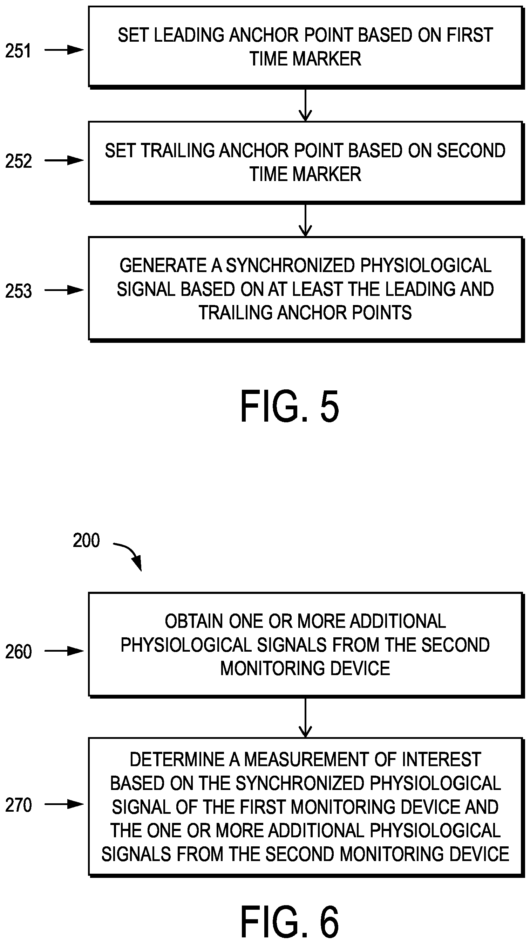

With reference to FIG. 5, the step 250 of synchronizing the first and second monitoring devices 102, 104 can include: in a step 251, setting a leading anchor point for at least one physiological signal 108 collected by the second monitoring device 104 and a leading anchor point for at least one physiological signal 106 collected by the first monitoring device 102, wherein the leading anchor points are set based on the first time marker; in a step 252, setting a trailing anchor point for the at least one physiological signal 108 collected by the second monitoring device 104 and a trailing anchor point for the at least one physiological signal 106 collected by the first monitoring device 102, wherein the trailing anchor points are set based on the second time marker; and in a step 253, generating a synchronized physiological signal.

In particular embodiments, the synchronized physiological signal may be generated resampling the at least one physiological signal 106 collected by the first monitoring device 102 based on the known (or nominal) sampling rate of the second monitoring device 104 and/or a relationship between the leading and trailing anchor points. In further embodiments, generating the synchronized physiological signal may include producing a common time-base for two or more un-synchronized signals 106, 108 in which the signals 106, 108 can be expressed in starting from and/or centered around a common moment in time (e.g., *t*₀). Put another way, synchronizing the physiological signals 106, 108 in the step 253 can include generating a mapping of the physiological signals 106, 108 to a common time-base based on at least the leading and trailing anchor points. In such embodiments, the mapping associates samples of every signal collected by the devices 102, 104 to that common time-base.

As a result, the physiological signals 106, 108 collected by the first monitoring device 102 will then be synchronized in timing and pacing with the second monitoring device 104, even though the first and second monitoring devices 102, 104 have separate electronic clocks. The first and second monitoring devices 102, 104 may remain synchronized for at least a period of time following the second period of time. That is, once the devices 102, 104 are synchronized, the physiological signals 106, 108 produced by those devices 102, 104 may remain synchronized for a period of time after the synchronization operation. In particular embodiments, because each physiological signal 106 collected by the first monitoring device 102 is derived from the same clock, each physiological signal 106 collected by the first monitoring device 102 may be synchronized with the signals 108 collected by the second monitoring device 104.

In embodiments, the method 200 can further include integrating one or more signals from the first and second monitoring devices 102, 104 after the devices 102, 104 are synchronized. In particular embodiments, one or more additional physiological parameters may be determined by integrating signals from both monitoring devices 102, 104.

For example, a PPG signal obtained from the second device 104 may be analyzed in conjunction with a synchronized ECG signal obtained from the first device 102 in order to determine a pulse arrival time. In another example, two PPG signals may be obtained from the devices 102, 104 in order to determine a pulse transit time.

As described herein, it is contemplated that each of the physiological sensors of a particular device 102, 104 are synchronized to a single clock (i.e., each of the physiological signals produced by one monitoring device are synchronized with each other). In particular embodiments, it is also contemplated that a monitoring device 102, 104 may be physiological sensors that are not synchronized, in which case the method 200 may include synchronizing two or more physiological signals of a single device 102, 104. In some embodiments, this may be accomplished, for example, by tapping, shaking, turning, and/or moving two devices together in a given pattern to create a recognizable accelerometry template. In other embodiments, the synchronization can be accomplished by providing photodiodes for the devices, which can then be illuminated with a predetermined pattern by an external LED light source or occluded by the user, by hand.

More generally, as shown in FIG. 6, the method 200 can include: in a step 260, obtaining one or more additional signals from at least one of the monitoring device 102, 104; and in a step 270, analyzing a synchronized physiological signal with the one or more additional signals to determine a measurement-of-interest. More specifically, the steps 260 and 270 can include obtaining at least a third physiological signal from at least one of the monitoring devices 102, 104 and analyzing the third physiological signal along with the synchronized physiological signal(s) in order to determine a physiological measurement-of-interest.

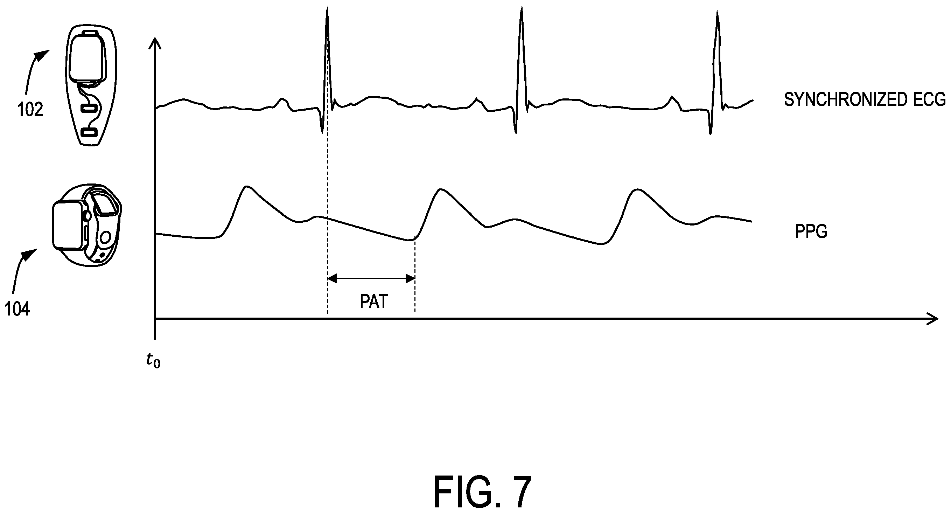

As mentioned above, the measurement-of-interest can be a pulse arrival time (PAT) and/or a pulse transit time (PTT). As shown in FIG. 7, a synchronized signal (such as a synchronized ECG signal) can be more readily compared with an additional signal (such as a PPG signal) in order to determine an accurate measurement-of-interest (such as a pulse arrival time) because the signals will be aligned and have corresponding sample frequencies. In particular, when the devices sample at sample rates that bear a different pace, a measure like transit time will change every next measurement due to that difference in pace.

Returning to FIG. 2, the method 200 can also include repeating one or more steps as shown. In some embodiments, the method 200 can include repeating steps 210-250 and/or steps 230-250 one or more times in order to check and maintain synchronized signals. For example, in particular embodiments, after synchronizing the first and second monitoring devices 102, 104 for a first time, the method 200 can include re-synchronizing the first and second monitoring devices 102, 104 again after a certain period of time has elapsed since the previous synchronization. In this manner, the two or more monitoring devices 102, 104 may be repeatedly synchronized so that shared signals may be continuously integrated and analyzed. In embodiments, the synchronization operation may be repeated every hour or several hours, every day or several days, every week or several weeks, and/or on-demand.

In particular embodiments, the method 200 may include a step 259 of analyzing the physiological signals 106, 108 after they are synchronized (e.g., resampled and/or expressed in a common time-base), in order to determine a third time marker indicative of a time lag between the synchronized signals. Put another way, a cross-correlation operation may be performed in the step 259 using the synchronized signals in order to confirm proper alignment of the synchronized signals. In such embodiments, if a time lag is found, the synchronized signals may be adjusted around the third time marker.

As described herein, the method 200 may be repeated one or more times to ensure synchronization between two or more devices 102, 104. It is contemplated that certain events or conditions may trigger the performance of the method 200, including, for example, when a device (e.g., device 102 or device 104) is taken off and put back on. In embodiments, other events or conditions may trigger the performance of the method 200, such as losing access to a device, if the temperature changes significantly (which may change the clock / crystal frequency of the electronics), or if abnormal signals are produced.

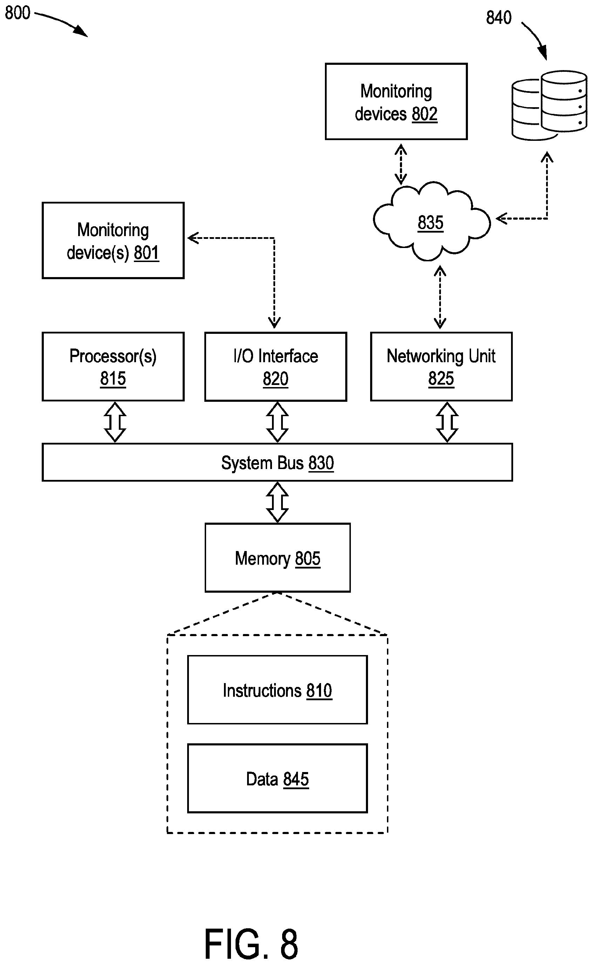

Turning now to FIG. 8, an exemplary health monitoring system 800 is illustrated according to certain aspects of the present disclosure. The health monitoring system 800 can be configured or adapted to synchronize separate health monitoring devices for accurate processing of shared signals, as described herein. In embodiments, the health monitoring system 800 can include a computer-readable storage medium 805 having stored thereon machine-readable instructions 810 to be executed by one or more processors 815, one or more processors 815 that are configured by the machine-readable instructions 810 stored on the computer-readable storage medium 805 to perform one or more steps of the methods described herein. In particular embodiments, the health monitoring system 800 can further include one or more health monitoring devices 801, 802 associated with one or more individuals. In the example of FIG. 8, the health monitoring system 800 also comprises an input/output interface 820 and/or a networking unit 825. As shown, the memory 805, the one or more processors 815, the input/output interface 820, and the networking unit 825 may be interconnected and/or communicate through a system bus 830 containing conductive circuit pathways through which instructions (e.g., machine-readable signals) and data may travel to effectuate communication, tasks, storage, and the like.

The one or more processors 815 may include one or more high-speed data processors adequate to execute the program components described herein and/or perform one or more steps of the methods described herein. The one or more processors 815 may include a microprocessor, a multi-core processor, a multithreaded processor, an ultra-low voltage processor, an embedded processor, and/or the like, including combinations thereof. The one or more processors 815 may include multiple processor cores on a single die and/or may be a part of a system on a chip (SoC) in which the processor 815 and other components are formed into a single integrated circuit, or a single package. As a non-exhaustive list, the one or more processors 815 can include one or more of an Intel^{®} Architecture Core^{™} based processor, such as a Quark^{™}, an Atom^{™}, an i3, an i5, and i7, or an MCU-class processor, an Advanced Micro Devices, Inc. (AMD) processor such as a Ryzen or Epyc based processor, an A5-A10 processor from Applet Inc., a Snapdragon^{™} processor from Qualcomm^{®} Technologies, Inc., and/or the like.

The input/output (I/O) interface 820 of the health monitoring system 800 may include one or more I/O ports that provide a physical connection to one or more devices, such as one or more health monitoring devices 801. Put another way, the I/O interface 820 may be configured to connect one or more health monitoring devices 801 of the health monitoring system 800 in order to facilitate communication and/or control of between such devices. In some embodiments, the I/O interface 820 can comprise one or more serial ports.

The networking unit 825 of the health monitoring system 800 may include one or more types of networking interfaces that facilitate wireless communication between one or more components of the health monitoring system 800 and/or between the health monitoring system 800 and one or more external components. In embodiments, the networking unit 825 may operatively connect the health monitoring system 800 to a communications network 835, which can include a direct interconnection, the Internet, a local area network ("LAN"), a metropolitan area network ("MAN"), a wide area network ("WAN"), a wired or Ethernet connection, a wireless connection, a cellular network, and similar types of communications networks, including combinations thereof. In some examples, health monitoring system 800 may communicate with one or more remote / cloud-based servers (e.g., electronic medical records database 840), cloud-based services, wired and/or wireless devices (e.g., wireless health monitoring devices 802) via the networking unit 825.

The memory 805 of the health monitoring system 800 can be variously embodied in one or more forms of machine-accessible and machine-readable memory. In some examples, the memory 805 may comprise one or more types of memory, including one or more types of transitory and/or non-transitory memory. In particular embodiments, the memory 805 can comprise a magnetic disk storage device, an optical disk storage device, an array of storage devices, a solid-state memory device, and/or the like, including combinations thereof. The memory 805 may also include one or more other types of memory, such as dynamic random-access memory (DRAM), static random-access memory (SRAM), erasable programmable read-only memory (EPROM), electrically erasable programmable read-only memory (EEPROM), Flash memory, and/or the like.

In particular embodiments, the memory 805 can be configured to store data 845 and machine-readable instructions 810 that, when executed by the one or more processors 815, cause the health monitoring system 800 to perform one or more steps of the methods and/or processes described herein. In some embodiments, the instructions 810 can include a synchronization module (e.g., synchronization module 114) configured to synchronize the physiological signals of two or more monitoring devices 102, 104, and/or an analysis module (e.g., analysis module 116) configured to integrate information using the synchronized signals.

More specifically, as described above, the memory 805 may include instructions 810 that, when executed by the one or more processors 815, cause the health monitoring system 800 to perform at least the following operations: machine-readable instructions stored on the computer-readable storage medium to perform the following operations: (i) obtain a first set of physiological signals from a first monitoring device and a second monitoring device, wherein the first set of physiological signals includes a first physiological signal from the first monitoring device covering a first time period and a second physiological signal from the second monitoring device covering at least a portion of the first time period; (ii) analyze at least the first and second physiological signals of the first set of physiological signals to determine a first time marker; (iii) obtain a second set of physiological signals from the first monitoring device and the second monitoring device, wherein the second set of physiological signals includes a first physiological signal from the first monitoring device covering a second time period and a second physiological signal from the second monitoring device covering at least a portion of the second time period; (iv) analyze at least the first and second physiological signals of the second set of physiological signals to determine a second time marker; and (v) synchronize the first and second monitoring devices based on the first and second time markers.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used herein, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept. Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively. It should also be understood that, unless clearly indicated to the contrary, in any methods claimed herein that include more than one step or act, the order of the steps or acts of the method is not necessarily limited to the order in which the steps or acts of the method are recited.

The above-described examples of the described subject matter can be implemented in any of numerous ways. For example, some aspects can be implemented using hardware, software or a combination thereof. When any aspect is implemented at least in part in software, the software code can be executed on any suitable processor or collection of processors, whether provided in a single device or computer or distributed among multiple devices/computers.

The present disclosure can be implemented as a system, a method, and/or a computer program product at any possible technical detail level of integration. The computer program product can include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium comprises the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, comprising an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, comprising a local area network (LAN) or a wide area network (WAN), or the connection can be made to an external computer (for example, through the Internet using an Internet Service Provider). In some examples, electronic circuitry comprising, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions can be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions can also be stored in a computer readable storage medium that can direct a computer, a programmable data processing apparatus, and/or other devices to function in a particular manner, such that the computer readable storage medium having instructions stored therein comprises an article of manufacture comprising instructions which implement aspects of the function/act specified in the flowchart and/or block diagram or blocks.

The computer readable program instructions can also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various examples of the present disclosure. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant can be entitled.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A health monitoring system (100, 800) configured to synchronize separate monitoring devices (102, 104) for accurate processing of shared signals, the system (100, 800) comprising:
a computer-readable storage medium (805) having stored thereon machine-readable instructions (810) to be executed by one or more processors (805); and
one or more processors (815) configured by the machine-readable instructions (810) stored on the computer-readable storage medium (805) to perform the following operations:
obtain a first set of physiological signals from a first monitoring device (102) and a second monitoring device (104), wherein the first set of physiological signals includes a first physiological signal (106) from the first monitoring device (102) covering a first time period and a second physiological signal (108) from the second monitoring device (104) covering at least a portion of the first time period;
analyze at least the first and second physiological signals (106, 108) of the first set of physiological signals to determine a first time marker;
obtain a second set of physiological signals from the first monitoring device (102) and the second monitoring device (104), wherein the second set of physiological signals includes a first physiological signal (106) from the first monitoring device (102) covering a second time period and a second physiological signal (108) from the second monitoring device (104) covering at least a portion of the second time period;
analyze at least the first and second physiological signals (106, 108) of the second set of physiological signals to determine a second time marker; and
synchronize the first and second monitoring devices (102, 104) based on the first and second time markers.

2. The health monitoring system (100, 800) of claim 1, wherein the first time marker is determined by correlating one or more signal features of the first and second physiological signals (106, 108) of the first set of physiological signals, the first time marker being indicative of when a shared signal feature is observed in the first and second physiological signals (106, 108) of the first set of physiological signals, and
wherein the second time marker is determined by correlating one or more signal features of the first and second physiological signals (106, 108) of the second set of physiological signals, the second time marker being indicative of when a shared signal feature is observed in the first and second physiological signals (106, 108) of the second set of physiological signals.

3. The health monitoring system (100, 800) of claim 1, wherein the first and second physiological signals (106, 108) of the first set of physiological signals are electrocardiogram signals, and wherein the first and second physiological signals (106, 108) of the second set of physiological signals are electrocardiogram signals.

4. The health monitoring system (100, 800) of claim 1, wherein synchronizing the first and second monitoring devices (102, 104) includes:
setting a leading anchor point for at least one physiological signal (108) collected by the second monitoring device (104) and a leading anchor point for at least one physiological signal (106) collected by the first monitoring device (102), wherein the leading anchor points are set based on the first time marker;
setting a trailing anchor point for the at least one physiological signal (108) collected by the second monitoring device (104) and a trailing anchor point for the at least one physiological signal (106) collected by the first monitoring device (102), wherein the trailing anchor points are set based on the second time marker; and
generating a synchronized physiological signal by resampling the at least one physiological signal (106) collected by first monitoring device (102) based on a relationship between the leading and trailing anchor points.

5. The health monitoring system (100, 800) of claim 4, wherein the one or more processors (815) are further configured by the machine-readable instructions (810) stored on the computer-readable storage medium (805) to perform the following operations:
(i) obtain at least a third physiological signal (108) from the second monitoring device (104); and
(ii) analyze the third physiological signal (108) and the synchronized physiological signal to determine a physiological measurement of interest.

6. The health monitoring system (100, 800) of claim 5, wherein the third physiological signal (108) is a photoplethysmography signal, the synchronized physiological signal is either a photoplethysmography signal or an electrocardiogram signal, and the physiological measurement of interest is at least one of a pulse arrival time and a pulse transit time.

7. The health monitoring system (100, 800) of claim 1, further comprising:
a first monitoring device (102) and a second monitoring device (104) in communication with the one or
more processors (805), wherein the first monitoring device (102) is configured to measure one or more physiological signals (106) including at least a first signal type and the second monitoring device (104) is configured to measure one or more physiological signals (108) including at least the first signal type.

8. A non-transitory computer-readable storage medium (805) having stored thereon machine-readable instructions (810) that, when executed by one or more processors (805), cause the one or more processors (815) to perform operations comprising:
obtaining a first set of physiological signals from a first monitoring device and a second monitoring device, wherein the first set of physiological signals includes a first physiological signal from the first monitoring device covering a first time period and a second physiological signal from the second monitoring device covering at least a portion of the first time period;
analyzing at least the first and second physiological signals of the first set of physiological signals to determine a first time marker;
obtaining a second set of physiological signals from the first monitoring device and the second monitoring device, wherein the second set of physiological signals includes a first physiological signal from the first monitoring device covering a second time period and a second physiological signal from the second monitoring device covering at least a portion of the second time period;
analyzing at least the first and second physiological signals of the second set of physiological signals to determine a second time marker; and
synchronizing the first and second monitoring devices based on the first and second time markers.

9. A computer-implemented method (200) for synchronizing multiple monitoring devices in a health monitoring system (100, 800), the method (200) comprising:
obtaining (210) a first set of physiological signals from a first monitoring device and a second monitoring device, wherein the first set of physiological signals includes a first physiological signal from the first monitoring device covering a first time period and a second physiological signal from the second monitoring device covering at least a portion of the first time period;
analyzing (220) at least the first and second physiological signals of the first set of physiological signals to determine a first time marker;
obtaining (230) a second set of physiological signals from the first monitoring device and the second monitoring device, wherein the second set of physiological signals includes a first physiological signal from the first monitoring device covering a second time period and a second physiological signal from the second monitoring device covering at least a portion of the second time period;
analyzing (240) at least the first and second physiological signals of the second set of physiological signals to determine a second time marker; and
synchronizing (250) the first and second monitoring devices based on the first and second time markers.

10. The computer-implemented method (200) of claim 9, wherein the first time marker is determined by correlating one or more signal features of the first and second physiological signals of the first set of physiological signals, the first time marker being indicative of when a shared signal feature is observed in the first and second physiological signals of the first set of physiological signals, and
wherein the second time marker is determined by correlating one or more signal features of the first and second physiological signals of the second set of physiological signals, the second time marker being indicative of when a shared signal feature is observed in the first and second physiological signals of the second set of physiological signals.

11. The computer-implemented method (200) of claim 9, wherein the first and second physiological signals of the first set of physiological signals are electrocardiogram signals, and wherein the first and second physiological signals of the second set of physiological signals are electrocardiogram signals.

12. The computer-implemented method (200) of claim 9, wherein synchronizing the first and second monitoring devices includes:
setting (251) a leading anchor point for at least one physiological signal collected by the second monitoring device and a leading anchor point for at least one physiological signal collected by the first monitoring device, wherein the leading anchor points are set based on the first time marker;
setting (252) a trailing anchor point for the at least one physiological signal collected by the second monitoring device and a trailing anchor point for the at least one physiological signal collected by the first monitoring device, wherein the trailing anchor points are set based on the second time marker; and
generating (253) a synchronized physiological signal by resampling the at least one physiological signal collected by the first monitoring device based on a relationship between the leading and trailing anchor points.

13. The computer-implemented method (200) of claim 12, further comprising:
obtaining (260) a third physiological signal from the second monitoring device; and
analyzing (270) the third physiological signal and synchronized physiological signal to determine a physiological measurement of interest.

14. The computer-implemented method (200) of claim 13, wherein the third physiological signal is a photoplethysmography signal, the synchronized physiological signal is either a photoplethysmography signal or an electrocardiogram signal, and the physiological measurement of interest is at least one of a pulse arrival time and a pulse transit time.

15. The computer-implemented method (200) of claim 9, wherein the health monitoring system (100, 800) is a cloud-based system configured to wirelessly receive one or more physiological signals from the first and second monitoring devices.
